# EUROPEAN PATENT SPECIFICATION

(11) **EP 4 084 671 B1**
(45) Date of publication and mention of the grant of the patent: **08.11.2023**
(21) Application number: 20807439.3
(22) Date of filing: 20.11.2020
(51) Int. Cl.: A61B 5/00, G16H 50/00, A61B 5/374, A61B 5/372, G16H 50/70

(54) **SYSTEM FOR DETECTING AND CLASSIFYING SEGMENTS OF SIGNALS FROM EEG-RECORDINGS**
SYSTEM ZUM ERKENNEN UND KLASSIFIZIEREN VON SEGMENTEN VON SIGNALEN AUS EEG-AUFZEICHNUNGEN
SYSTÈME PERMETTANT DE DÉTECTER ET DE CLASSER DES SEGMENTS DE SIGNAUX À PARTIR D'ENREGISTREMENTS EEG

(30) Priority: 16.01.2020 EP 20152275
(43) Date of publication of application: 09.11.2022
(73) Proprietor: Prolira B.V., 3584 CH Utrecht (NL)
(72) Inventor: ZEMAN, Philip Michael, 3584 CH Utrecht (NL); VAN MERKERK, Rutger Olof, 3584 CH Utrecht (NL); VAN ZON, Arnout Tim, 3584 CH Utrecht (NL)
(74) Representative: IPecunia
(86) International application number: PCT/EP2020/082823
(87) International publication number: WO 2021/144053

(56) References cited:
- WO-A1-2020/002519
- US-A- 5 846 208
- US-A9- 2018 146 879
- T. NUMAN ET AL: "Delirium detection using relative delta power based on 1-minute single-channel EEG: a multicentre study", BRITISH JOURNAL OF ANAESTHESIA., vol. 122, no. 1, 1 January 2019 (2019-01-01), pages 60-68, XP055714483, GB ISSN: 0007-0912, DOI: 10.1016/j.bja.2018.08.021
- LASITHA S. VIDYARATNE ET AL: "Real-Time Epileptic Seizure Detection Using EEG", IEEE TRANSACTIONS ON NEURAL SYSTEMS AND REHABILITATIONENGINEERING., vol. 25, no. 11, 1 November 2017 (2017-11-01), pages 2146-2156, XP055715083, US ISSN: 1534-4320, DOI: 10.1109/TNSRE.2017.2697920
- SALEM OSMAN ET AL: "Epileptic seizure detection from EEG signal using Discrete Wavelet Transform and Ant Colony classifier", 2014 IEEE INTERNATIONAL CONFERENCE ON COMMUNICATIONS (ICC), IEEE, 10 June 2014 (2014-06-10), pages 3529-3534, XP032632215, DOI: 10.1109/ICC.2014.6883868 [retrieved on 2014-08-26]
- Giulia Pellegrino: "Analysis for Automatic Detection of Epileptic Seizure from EEG signals", , 14 July 2014 (2014-07-14), XP055715059, Retrieved from the Internet: URL:http://tesi.cab.unipd.it/46118/1/Thesi sBio.pdf [retrieved on 2020-07-16]
- Arendina Van Der Kooi: "Neurophysioly of delirium", , 1 January 2014 (2014-01-01), XP055714505, ISBN: 978-90-39-36141-2 Retrieved from the Internet: URL:https://prolira.com/wp-content/uploads /2020/06/vanderKooi.pdf [retrieved on 2020-07-14]
- OLIVER FAUST ET AL: "Wavelet-based EEG processing for computer-aided seizure detection and epilepsy diagnosis", SEIZURE, vol. 26, 24 January 2015 (2015-01-24), pages 56-64, XP055513018, GB ISSN: 1059-1311, DOI: 10.1016/j.seizure.2015.01.012
- OCAK ET AL: "Automatic detection of epileptic seizures in EEG using discrete wavelet transform and approximate entropy", EXPERT SYSTEMS WITH APPLICATIONS, OXFORD, GB, vol. 36, no. 2, 1 March 2009 (2009-03-01), pages 2027-2036, XP025680716, ISSN: 0957-4174, DOI: 10.1016/J.ESWA.2007.12.065 [retrieved on 2008-01-01]

## Description

### FIELD OF THE INVENTION

The invention relates to a system that is configured and arranged to perform a method for detecting and classifying a segment of a signal that is obtained from a single-channel EEG-recording as a target signal segment or as a non-target signal segment.

### BACKGROUND OF THE INVENTION

Delirium is an acute disturbance of consciousness and cognition that usually fluctuates over time. It is a common disorder, with reported incidences of more than 60% during Intensive Care Unit (ICU) stay and over 15% on a geriatric ward or medium care unit. Delirium is associated with higher mortality, longer hospitalization, long-term cognitive impairment and increased costs. There are three different subtypes of delirium based on psychomotor behavior, i.e. hypoactive, hyperactive and mixed-type delirium.

Despite its frequency and impact, recognition of delirium by health care professionals is poor. Exceptions are hyperactive forms of delirium, but these are relatively rare. Furthermore, delayed treatment of delirium in ICU patients was found to increase mortality. To improve early diagnosis and treatment, the Society of Critical Care Medicine and the American Psychiatric Association recommend daily monitoring of delirium in ICU patients.

Various delirium assessment tools have been developed including for example the Confusion Assessment Method for the ICU (CAM-ICU) as well as methods and systems that involve electroencephalography (EEG) using for example single-channel EEG-recordings.

The disclosure by T. Numan et al.: "Delirium detection using relative delta power based on 1-minute single-channel EEG: a multicentre study", British Journal of Anaesthesia, vol. 122, no. 1, 1 January 2019, pages 60-68 discloses delirium detection using an algorithm for EEG analyses based on spectral analysis. The algorithm provides a normalized delta power, the so-called relative delta power, by dividing the power in the so-called delta EEG frequency band (1-4Hz) and in the frequency band of 1-6Hz by the power in the total EEG frequency band of 1-30 Hz.

Document WO2020/002519A1 discloses a multi-class classification method for assessing quality of electroencephalographic (EEG) signals, which involves calculating distance between feature value of each channel of EEG signal segment and each feature value of training samples. The method involves receiving segments of EEG signal acquired from electrodes. The feature value is extracted from each channel of the EEG signal segments. A first classification is performed so as to assign each channel of the EEG signal segment to one of at least three quality classes. The first classification is performed by a k-nearest neighbours algorithm using a first training set comprising multiples training (reference) samples. Each of the training sample of the first training set is associated to one of the quality classes and to the feature value. The quality class which is the most frequent class among the k training samples of the first training set which are nearer to each channel of the EEG signal segment is assigned to each channel of the EEG signal segment. The distance is calculated between the feature value of each channel of the EEG signal segment and each feature value of the training samples.

A disadvantage of known delirium assessment methods and systems that use single-channel EEG-recordings is that they do not provide a reliable distinction between target signal segments and non-target signal segments of a single-channel EEG-recording, in particular when the target signal segments and the non-target signal segments of the single-channel EEG-recording look alike. It is noted that in the context of the present invention target signal segments of a single-channel EEG-recording are to be construed as signal segments that are, for example, indicative for a patient being delirious or suffering from related encephalopathy, whereas non-target signal segments of a single-channel EEG-recording are to be construed as signal segments that are indicative for non-target brain signals or artifacts such as for example eye artifacts, artifacts related to muscle activity, or artifacts related to a combination of such artifacts.

In view of the abovementioned disadvantage of known delirium assessment methods and systems using single-channel EEG-recordings, there is a need to provide a system that enables improved distinction between target signal segments and non-target signal segments of a single-channel EEG-recording.

### SUMMARY OF THE INVENTION

It is an object of the invention to provide a system to perform a method for detecting and classifying a segment of a signal that is obtained from a single-channel EEG-recording as a target signal segment or as a non-target signal segment that pre-empts or at least reduces the abovementioned disadvantage and/or other disadvantages associated with known delirium assessment methods using single-channel EEG-recordings.

Aspects of the present invention are set out in the accompanying independent and dependent claims. Features from the dependent claims may be combined with features from the independent claim as appropriate and not merely as explicitly set out in the claims.

At least one of the abovementioned objects is achieved by using in a system according to the invention, as defined by the claims, a data processing method for detecting and classifying a segment of a signal that is obtained from a single-channel EEG-recording as a target signal segment or as a non-target signal segment, the method comprising:
- providing a signal that is obtained from a single-channel EEG-recording;
- applying to said signal a target parameter set, which is indicative for a plurality of reference target signal segments that are obtained from reference single-channel EEG-recordings, to detect a first signal segment of said signal and to classify the detected first signal segment as a target signal segment, wherein the target parameter set comprises wavelet coefficients that are determined using wavelet decomposition of the plurality of reference target signal segments;
- assigning a first time stamp to the detected first signal segment;
- applying to said signal a non-target parameter set, which is indicative for a plurality of reference non-target signal segments that are obtained from reference single-channel EEG-recordings, to detect a second signal segment of said signal and to classify the detected second signal segment as a non-target signal segment, wherein the non-target parameter set comprises wavelet coefficients that are determined using wavelet decomposition of the plurality of reference non-target signal segments;
- assigning a second time stamp to the detected second signal segment;
- determining a temporal proximity of the first time stamp and the second time stamp;
- based on said determined temporal proximity, determining if a voting process is required to determine whether classification of the detected first signal segment as a target signal segment or classification of the detected second signal segment as a non-target signal segment is correct; and
- upon establishing that said voting process is required, performing said voting process.

In this way, an improved distinction between target signal segments and non-target signal segments of a signal that is obtained from a single-channel EEG-recording can be made and therefore false positive detections or incorrect classifications for that matter can be reduced.

The plurality of reference target signal segments and the plurality of reference non-target signal segments are obtained from signals that are obtained from single-channel EEG-recordings. The person skilled in the art will appreciate that single-channel EEG-recordings can be acquired in various known ways including for example using a differential electrode pair, using a single electrode in combination with a reference (REF) electrode or a ground (GND) electrode, or using a differential electrode pair in combination with a REF electrode or a GND electrode. In accordance with the latter exemplary electrode configuration, the individual electrodes of the differential electrode pair can be positioned at specific midline frontal, e.g. Fz, midline vertex, e.g. Cz, midline parietal, e.g. Pz, left medial temporal, e.g. T3, right medial temporal, e.g. T4, positions on a patient's scalp in accordance with an extended 10-20 EEG system with the REF electrode positioned, for example, on an ear of the patient. In addition to the above-mentioned examples for acquiring single-channel EEG-recordings, the person skilled in the art will appreciate that a single-channel EEG-recording in the context of the present invention can also be a single channel from a standard 10-20 EEG montage or from any other multichannel EEG montage for that matter. Regarding the latter interpretation of the phrase "single-channel EEG-recording", it is noted that in the context of the present invention, the plurality of reference target signal segments and the plurality of reference non-target signal segments are obtained exclusively from data of a single channel.

The single-channel EEG-recordings used to obtain the plurality of reference target signal segments and the plurality of reference non-target signal segments can have a predefined duration of for example 15 minutes. However, the person skilled in the art will appreciate that any suitable predefined duration can be used as long as the acquired single-channel EEG-recordings enable obtaining suitable reference target signal segments and reference non-target signal segments.

The reference target signal segments of the plurality of reference target signal segments can be mutually different. The same holds for the reference non-target signal segments of the plurality of non-target signal segments. The plurality of reference target signal segments can for example comprise more than 1000 reference target signal segments. The same holds for the plurality of non-target signal segments.

The target parameter set that is indicative for the plurality of reference target signal segments is determined by processing and analyzing the reference target signal segments of the plurality of reference target signal segments. The aforementioned processing and analyzing can be done using a training process that involves a machine learning algorithm that can for example use deep neural networks. The processing and analyzing can for example be done in the frequency domain. The target parameter set can be construed as an aggregate parameter set as it comprises parameters that are indicative for the plurality of reference target signal segments.

Upon determining the target parameter set, it is used to detect a first signal segment of a single-channel EEG-recording that is acquired in one of the above-mentioned ways and to classify the detected first signal segment as a target signal segment, i.e. a signal segment that is indicative for a patient being delirious or suffering from related encephalopathy. Upon detecting the first signal segment, it is marked by assigning the first time stamp to it.

In an analogous way, the non-target parameter set that is indicative for the plurality of reference non-target signal segments is determined by processing and analyzing the reference non-target signal segments of the plurality of reference non-target signal segments. The aforementioned processing and analyzing can be done using another training process that involves another machine learning algorithm that can for example use deep neural networks. The processing and analyzing can for example be done in the frequency domain. The non-target parameter set can be construed as an aggregate parameter set as it comprises parameters that are indicative for the plurality of reference non-target signal segments.

Upon determining the non-target parameter set, it is used to detect a second signal segment of the same signal that was obtained from the single-channel EEG-recording that is acquired in one of the above-mentioned ways and to classify the detected second signal segment as a non-target signal segment, i.e. a signal segment that is indicative for artifacts such as for example eye artifacts, artifacts related to muscle activity, or artifacts related to a combination of such artifacts. Upon detecting the second signal segment, it is marked by assigning the second time stamp to it.

In accordance with the present invention, determining a temporal proximity of the first time stamp and the second time stamp involves determining a time difference between the first time stamp and the second time stamp. The determined time difference is compared with a predefined threshold. The person skilled in the art will appreciate that the predefined threshold is chosen such that when the determined time difference between the first time stamp and the second time stamp is smaller than the threshold, it can be not likely or not possible at all that the classification of the detected first signal segment as being a target signal segment and the classification of the detected second signal segment as being a non-target signal segment can both be correct. Hence, when the determined time difference is smaller than the predefined threshold the voting process is required to determine whether classification of the detected first signal segment as a target signal segment or the classification of the detected second signal segment as a non-target signal segment is correct. If the determined time difference is equal to or larger than the threshold then the classification of the detected first signal segment as being a target signal segment and the classification of the detected second signal segment as being a non-target signal segment are most likely both correct.

The person skilled in the art will appreciate that any suitable predefined threshold can be chosen as long as it allows to establish whether the classification of the detected first signal segment as being a target signal segment and/or the classification of the detected second signal segment as being a non-target signal segment can be correct. Suitable values for the predefined threshold range between 0.25 s and 3 s. Preferably, the threshold is 1 s.

Based on the above, it will be clear that the voting process eliminates one of the classifications. As a result, the system according to the present invention can reduce false positive detections or incorrect classifications for that matter.

Based on the above, an example of the data processing method for use in a system according to the present invention (the method, per se, not being part of the claimed invention) is a method for detecting and classifying a segment of a signal that is obtained from a single-channel EEG-recording as a target signal segment or as a non-target signal segment, the method comprising:
- providing a signal that is obtained from a single-channel EEG-recording;
- applying to said signal a target parameter set that is indicative for a plurality of reference target signal segments that are obtained from reference single-channel EEG-recordings to detect a first signal segment and to classify the detected first signal segment as a target signal segment;
- assigning a first time stamp to the detected first signal segment;
- applying to said signal a non-target parameter set that is indicative for a plurality of reference non-target signal segments that are obtained from reference single-channel EEG-recordings to detect a second signal segment and to classify the detected second signal segment as a non-target signal segment;
- assigning a second time stamp to the detected second signal segment;
- determining a temporal proximity of the first time stamp and the second time stamp;
- based on said determined temporal proximity, determining if a voting process is required to determine whether classification of the detected first signal segment as a target signal segment or classification of the detected second signal segment as a non-target signal segment is correct; and
- upon establishing that said voting process is required, performing said voting process.

In an embodiment of the method for use in a system according to the invention, performing the voting process comprises:
- generating a first signal sample that comprises the detected first signal segment;
- matching the first signal sample with the plurality of reference target signal segments to determine a best target match;
- generating a second signal sample that comprises the detected second signal segment;
- matching the second signal sample with the plurality of reference non-target signal segments to determine a best non-target match;
- applying metrics to the first signal sample, the best target match, the second signal sample and the best non-target match to determine:
   - whether the classification of the detected first signal segment as a target signal segment is correct; or
   - whether the classification of the detected second signal segment as a non-target signal segment is correct.

In accordance with the present disclosure matching the first signal sample with the plurality of reference target signal segments to determine the best target match can involve for example curve fitting in the time domain of the first signal sample with the plurality of reference target signal segments. In an analogous way, matching the second signal sample with the plurality of reference non-target signal segments to determine the best non-target match can involve for example curve fitting in the time domain of the second signal sample with the plurality of reference non-target signal segments.

In accordance with the present disclosure curve fitting in the time domain may include comparing the signal shape of the first signal sample with the signal shapes of the reference target signal segments of the plurality of reference target signal segments and comparing of the signal shape of the second signal sample with the signal shapes of the reference non-target signal segments of the plurality of reference non-target signal segments. In this case the curve fit resulting in for example the smallest residue can be chosen to determine the best target match and the best non-target match, respectively. However, other aspects related to the curve fitting process can of course also be regarded to determine the best target match and the best non-target match, respectively.

The person skilled in the art will appreciate that curve fitting in the time domain is just an example of the analysis methods that are available to determine the best target match and the best non-target match, respectively. Examples of analysis methods include for example Fast Fourier Transform (FFT), linear signal analysis techniques involving determination of coherence, non-linear signal analysis techniques involving determination of phase synchronization and/or generalized synchronization, template matching, and parametric models including the use of wavelets.

In accordance with the present invention, applying metrics to the first signal sample, the best target match, the second signal sample and the best non-target match can be done in several different ways. A first way of doing this is by establishing and comparing a correlation in the time domain. A second way of doing this is by establishing and comparing a goodness of fit in the wavelet domain. By applying either one of these techniques it can be determined whether the classification of the detected first signal segment as being a target signal segment or the classification of the detected second signal segment as being a non-target signal segment is correct.

As a result of the above, it will be clear that the voting process can eliminate one of the two classifications and thereby will determine the final classification as target signal segment or as non-target signal segment. As a result, false positive classifications or incorrect detections for that matter can be reduced.

In an embodiment of the method for use in a system according to the invention (the method, per se, not being part of the claimed invention) performing the voting process comprises:
- generating a first signal sample that comprises the detected first signal segment;
- matching the first signal sample with a set of reference target signal segments that is based on the plurality of reference target signal segments to determine a best target match;
- generating a second signal sample that comprises the detected second signal segment;
- matching the second signal sample with a set of reference non-target signal segments that is based on the plurality of reference non-target signal segments to determine a best non-target match;
- applying metrics to the first signal sample, the best target match, the second signal sample and the best non-target match to determine:
   - whether the classification of the detected first signal segment as a target signal segment is correct; or
   - whether the classification of the detected second signal segment as a non-target signal segment is correct.

The person skilled in the art will appreciate that the same considerations as mentioned above regarding the previous embodiment of the present disclosure equally apply to the steps of matching the first signal sample and the second signal sample with the set of reference target signal segments that is based on the plurality of reference target signal samples, and the set of reference non-target signal segments that is based on the plurality of reference non-target signal samples of the currently mentioned embodiment of the present invention.

Moreover, the person skilled in the art will appreciate that the same considerations as mentioned above regarding the previous embodiment of the present disclosure equally apply to the step of applying metrics to the first signal sample, the best target match, the second signal sample and the best non-target match of the currently mentioned embodiment of the present invention.

Furthermore, it will be clear that by using a set of reference target signal segments that is based on the plurality of reference target signal segments and a set of reference non-target signal segments that is based on the plurality of reference non-target signal segments the voting process can eliminate one of the two classifications without having to use all reference target signal segments of the plurality of reference target signal segments and all reference non-target signal segments of the plurality of reference non-target signal segments, respectively. As a result, the voting process can be performed faster.

In an embodiment of the method for use in a system according to the invention (the method itself not being part of the claimed invention) the method further comprises removing the classification of the detected first signal segment or the classification of the detected second signal segment that based on the voting process is incorrect. The person skilled in the art will appreciate that the voting process used in a system according to the present invention results in a so-called winner, i.e. either the classification of the detected first signal segment as being a target signal segment or the classification of the detected second signal segment as being a non-target signal segment is correct. The classification that in accordance with the voting process is to be regarded as incorrect will be removed.

In an embodiment of the method for use in a system according to the invention (the method itself not being part of the claimed invention) a predetermined detection boundary, which is determined based on the target parameter set and/or the non-target parameter set, is applied that allows classification of detected signal segments as target signal segments or as non-target signal segments. The detection boundary can for example be a boundary plane in the feature space of wavelet coefficients that is determined by training on a labeled training set of target signal segments and non-target signal segments. In this way, another way to make a distinction between target signal segments and non-target signal segments of a single-channel EEG-recording can be provided. Hence, false positive detections or incorrect classifications for that matter can be reduced.

In an example of the method for use in a system according to the invention (the method itself not being part of the claimed invention), based on the target parameter set and/or the non-target parameter set a detection boundary is determined that allows classification of detected signal segments as target signal segments or as non-target signal segments.

In an embodiment of the method for use in a system according to the invention (the method itself not being part of the claimed invention), the target parameter set comprises wavelet coefficients that are determined using wavelet decomposition of the plurality of reference target signal segments, and the non-target parameter set comprises wavelet coefficients that are determined using wavelet decomposition of the plurality of reference non-target signal segments.

The wavelet coefficients are determined by wavelet decomposition of the reference target signal segments of the plurality of reference target signal segments and of the reference non-target signal segments of the plurality of the reference non-target signal segments. Upon determining the respective wavelet coefficients for the reference target signal segments and for the reference non-target signal segments, a training process that involves a machine learning algorithm can be used to identify the wavelet coefficients that are most representative for the reference target signal segments and for the reference non-target signal segments, respectively. The machine learning algorithm can for example use deep neural networks. The person skilled in the art will appreciate that the target parameter set preferably comprises the wavelet coefficients that are most representative for the reference target signal segments and that the non-target parameter set preferably comprises the wavelet coefficients that are most representative for the reference non-target signal segments. The person skilled in the art will appreciate that the thus obtained target parameter set and the non-target parameter set can comprise statistical average values of the wavelet coefficients that are most representative for the plurality of reference target signal segments and for the plurality of reference non-target signal segments, respectively.

In an embodiment of the method for use in a system according to the invention (the method itself not being part of the claimed invention), the method further comprises determining an optimized target parameter set that comprises wavelet coefficients that are indicative specifically for the plurality of reference target signal segments and/or an optimized non-target parameter set that comprises wavelet coefficients that are indicative specifically for the plurality of reference non-target signal segments.

Determination of the optimized target parameter set and the optimized non-target parameter set can be achieved in several ways. A first way of doing this involves comparing of the wavelet coefficients of the target parameter set and the wavelet coefficients of the non-target parameter set, wherein wavelet coefficients that occur both in the target parameter set and in the non-target parameter set are removed from the target parameter set and/or from the non-target parameter set. In this way, overlap between the target parameter set and the non-target parameter set can be reduced.

A second way of determining the optimized target parameter set and the optimized non-target parameter set involves using the resulting sensitivity and specificity of a system, e.g. a classifier unit, that is adapted to classify a detected first signal segment as being a target signal segment and a detected second signal segment as being a non-target signal segment.

As a result of any one of the above-mentioned first way and second way, the thus optimized target parameter set and non-target parameter set enable an improved distinction between target signal segments and non-target signal segments of a single-channel EEG-recording. Hence, false positive detections or incorrect classifications for that matter can be reduced.

In an embodiment of the method for use in a system according to the invention (the method itself not being part of the claimed invention), based on the optimized target parameter set and/or the optimized non-target parameter set a detection boundary is determined that allows improved classification of detected signal segments as target signal segments or as non-target signal segments. In this way, an improved distinction between target signal segments and non-target signal segments of a single-channel EEG-recording can be achieved. Hence, false positive detections or incorrect classifications for that matter can be reduced.

According to another aspect, a device is provided that is configured and arranged to be used with the system according to the invention that is configured and arranged to detect and classify a segment of a signal that is obtained from a single-channel EEG-recording as a target signal segment or as a non-target signal segment, the device having a database comprising at least one of:
- a plurality of reference target signal segments that are obtained from reference single-channel EEG-recordings;
- a set of reference target signal segments that is based on the plurality of reference target signal segments;
- a plurality of reference non-target signal segments that are obtained from reference single-channel EEG-recordings;
- a set of reference non-target signal segments that is based on the plurality of reference non-target signal segments;
- a target parameter set that is indicative for the plurality of reference target signal segments; and
- a non-target parameter set that is indicative for a plurality of reference non-target signal segments.

The device can be construed as a detector that comprises dedicated parameter sets, i.e. the target parameter set that is indicative for the plurality of reference target signal segments and the non-target parameter set that is indicative for the plurality of reference non-target signal segments, wherein the dedicated parameter sets can be obtained via respective training processes as described above that may involve respective machine learning algorithms that may use respective deep neural networks.

The device enables an improved distinction between target signal segments and non-target signal segments of a signal that is obtained from a single-channel EEG-recording. As a result, false positive detections or incorrect classifications for that matter as discussed above can be reduced.

According to the present invention, a system is provided that is configured and arranged to detect and classify a segment of a signal that is obtained from a single-channel EEG-recording as a target signal segment or as a non-target signal segment, the system comprising a processor that is configured and arranged to perform the method for use in a system according to the present invention on said signal when being operatively connected to the device for use in the system according to the present invention.

In this way, the system and the device when being operatively connected can be used to achieve an improved distinction between target signal segments and non-target signal segments of a signal that is obtained from a single-channel EEG-recording. As a result, false positive detections or incorrect classifications for that matter as discussed above can be reduced. The person skilled in the art will appreciate that the device and the system can be implemented as separate units. However, the device and the system can also be implemented as an integrated unit.

According to the present invention, a system is provided that is configured and arranged to detect and classify a segment of a signal that is obtained from a single-channel EEG-recording as a target signal segment or as a non-target signal segment, the system comprising a processor that is configured and arranged to perform on said signal when being operatively connected to a device having a database, the process steps of:
- providing a signal that is obtained from a single-channel EEG-recording;
- applying to said signal a target parameter set, which is indicative for a plurality of reference target signal segments that are obtained from reference single-channel EEG-recordings, to detect a first signal segment of said signal and to classify the detected first signal segment as a target signal segment, wherein the target parameter set comprises wavelet coefficients that are determined using wavelet decomposition of the plurality of reference target signal segments;
- assigning a first time stamp to the detected first signal segment;
- applying to said signal a non-target parameter set, which is indicative for a plurality of reference non-target signal segments that are obtained from reference single-channel EEG-recordings, to detect a second signal segment of said signal and to classify the detected second signal segment as a non-target signal segment, wherein the non-target parameter set comprises wavelet coefficients that are determined using wavelet decomposition of the plurality of reference non-target signal segments;
- assigning a second time stamp to the detected second signal segment;
- determining a time difference between the first time stamp and the second time stamp;
- when said determined time difference is smaller than a predetermined threshold, determining if a voting process is required to determine whether classification of the detected first signal segment as a target signal segment or classification of the detected second signal segment as a non-target signal segment is correct; and
upon establishing that said voting process is required, performing said voting process, wherein the database (4) comprises at least one of:
- the plurality of reference target signal segments that are obtained from reference single-channel EEG-recordings;
- a set of reference target signal segments that is based on the plurality of reference target signal segments;
- the plurality of reference non-target signal segments that are obtained from reference single-channel EEG-recordings;
- a set of reference non-target signal segments that is based on the plurality of reference non-target signal segments;
- the target parameter set that is indicative for the plurality of reference target signal segments, wherein the target parameter set comprises wavelet coefficients that are determined using wavelet decomposition of the plurality of reference target signal segments; and
- the non-target parameter set that is indicative for a plurality of reference non-target signal segments, wherein the non-target parameter set comprises wavelet coefficients that are determined using wavelet decomposition of the plurality of reference non-target signal segments.

In this way, an improved distinction between target signal segments and non-target signal segments of a signal that is obtained from a single-channel EEG-recording can be made and therefore false positive detections or incorrect classifications for that matter can be reduced.

The plurality of reference target signal segments and the plurality of reference non-target signal segments are obtained from signals that are obtained from single-channel EEG-recordings. The person skilled in the art will appreciate that single-channel EEG-recordings can be acquired in various known ways including for example using a differential electrode pair, using a single electrode in combination with a reference (REF) electrode or a ground (GND) electrode, or using a differential electrode pair in combination with a REF electrode or a GND electrode. In accordance with the latter exemplary electrode configuration, the individual electrodes of the differential electrode pair can be positioned at specific midline frontal, e.g. Fz, midline vertex, e.g. Cz, midline parietal, e.g. Pz, left medial temporal, e.g. T3, right medial temporal, e.g. T4, positions on a patient's scalp in accordance with an extended 10-20 EEG system with the REF electrode positioned, for example, on an ear of the patient. The single-channel EEG-recordings used to obtain the plurality of reference target signal segments and the plurality of reference non-target signal segments can have a predefined duration of for example 15 minutes. However, the person skilled in the art will appreciate that any suitable predefined duration can be used as long as the acquired single-channel EEG-recordings enable obtaining suitable reference target signal segments and reference non-target signal segments.

The reference target signal segments of the plurality of reference target signal segments can be mutually different. The same holds for the reference non-target signal segments of the plurality of non-target signal segments. The plurality of reference target signal segments can for example comprise more than 1000 reference target signal segments. The same holds for the plurality of non-target signal segments.

The target parameter set that is indicative for the plurality of reference target signal segments can be determined by processing and analyzing the reference target signal segments of the plurality of reference target signal segments. The aforementioned processing and analyzing can be done using a training process that involves a machine learning algorithm that can for example use deep neural networks. The processing and analyzing can for example be done in the frequency domain. The target parameter set can be construed as an aggregate parameter set as it comprises parameters that are indicative for the plurality of reference target signal segments. The target parameter set comprises wavelet coefficients that are determined using wavelet decomposition of the plurality of reference target signal segments.

Upon determining the target parameter set, it is be used to detect a first signal segment of a single-channel EEG-recording that is acquired in one of the above-mentioned ways and to classify the detected first signal segment as a target signal segment, i.e. a signal segment that is indicative for a patient being delirious or suffering from related encephalopathy. Upon detecting the first signal segment, it is marked by assigning the first time stamp to it.

In an analogous way, the non-target parameter set that is indicative for the plurality of reference non-target signal segments is determined by processing and analyzing the reference non-target signal segments of the plurality of reference non-target signal segments. The aforementioned processing and analyzing can be done using another training process that involves another machine learning algorithm that can for example use deep neural networks. The processing and analyzing can for example be done in the frequency domain. The non-target parameter set can be construed as an aggregate parameter set as it comprises parameters that are indicative for the plurality of reference non-target signal segments. The non-target parameter set comprises wavelet coefficients that are determined using wavelet decomposition of the plurality of reference non-target signal segments.

The wavelet coefficients can be determined by wavelet decomposition of the reference target signal segments of the plurality of reference target signal segments and of the reference non-target signal segments of the plurality of the reference non-target signal segments. Upon determining the respective wavelet coefficients for the reference target signal segments and for the reference non-target signal segments, a training process that involves a machine learning algorithm can be used to identify the wavelet coefficients that are most representative for the reference target signal segments and for the reference non-target signal segments, respectively. The machine learning algorithm can for example use deep neural networks. The person skilled in the art will appreciate that the target parameter set preferably comprises the wavelet coefficients that are most representative for the reference target signal segments and that the non-target parameter set preferably comprises the wavelet coefficients that are most representative for the reference non-target signal segments. The person skilled in the art will appreciate that the thus obtained target parameter set and the non-target parameter set can comprise statistical average values of the wavelet coefficients that are most representative for the plurality of reference target signal segments and for the plurality of reference non-target signal segments, respectively.

Upon determining the non-target parameter set, it is used to detect a second signal segment of the same signal that was obtained from the single-channel EEG-recording that is acquired in one of the above-mentioned ways and to classify the detected second signal segment as a non-target signal segment, i.e. a signal segment that is indicative for artifacts such as for example eye artifacts, artifacts related to muscle activity, or artifacts related to a combination of such artifacts. Upon detecting the second signal segment, it can be marked by assigning the second time stamp to it.

In accordance with the present disclosure determining a temporal proximity of the first time stamp and the second time stamp can be done in several different ways.

A first exemplary way of doing this involves dividing the obtained first signal in time intervals of a predefined length. Preferably, the time intervals have a predefined equal length. These time intervals can be referred to as bins. Preferably, the time intervals or bins have a predefined equal length. The predefined length of the time intervals can be chosen depending on specific requirements such as desired accuracy. Suitable predefined lengths of the time intervals range between 0.25 s and 3 s. Preferably, the time intervals have a predefined length of 1 s. Determining the temporal proximity of the first time stamp and the second time stamp is based on establishing if the first time stamp and the second time stamp fall within the same time interval or not. If the first time stamp and the second time stamp fall within the same time interval or bin, then the voting process is required and will be performed to determine whether classification of the detected first signal segment as a target signal segment or classification of the detected second signal segment as a non-target signal segment is correct. If the first time stamp and the second time stamp fall in different time intervals, i.e. the first time stamp and the second time stamp do not fall within the same bin, then the voting process is not required and preferably the voting process is not performed. In this case, the classification of the detected first signal segment as being a target signal segment and the classification of the detected second signal segment as being a non-target signal segment are most likely both correct.

A second way of determining the temporal proximity of the first time stamp and the second time stamp, according to the claimed invention, involves determining a time difference between the first time stamp and the second time stamp. The determined time difference is compared with a predefined threshold. The person skilled in the art will appreciate that the predefined threshold is chosen such that when the determined time difference between the first time stamp and the second time stamp is smaller than the threshold, it can be not likely or not possible at all that the classification of the detected first signal segment as being a target signal segment and the classification of the detected second signal segment as being a non-target signal segment can both be correct. Hence, when the determined time difference is smaller than the predefined threshold the voting process is required to determine whether classification of the detected first signal segment as a target signal segment or the classification of the detected second signal segment as a non-target signal segment is correct. If the determined time difference is equal to or larger than the threshold then the classification of the detected first signal segment as being a target signal segment and the classification of the detected second signal segment as being a non-target signal segment are most likely both correct.

The person skilled in the art will appreciate that any suitable predefined threshold can be chosen as long as it allows to establish whether the classification of the detected first signal segment as being a target signal segment and/or the classification of the detected second signal segment as being a non-target signal segment can be correct. Suitable values for the predefined threshold range between 0.25 s and 3 s. Preferably, the threshold is 1 s.

Based on the above, it will be clear that the voting process eliminates one of the classifications. As a result, the system according to the present invention can reduce false positive detections or incorrect classifications for that matter.

In an embodiment of the system according to the disclosure the processor is configured and arranged to perform the voting process comprising the process steps of:
- generating a first signal sample that comprises the detected first signal segment;
- matching the first signal sample with the plurality of reference target signal segments to determine a best target match;
- generating a second signal sample that comprises the detected second signal segment;
- matching the second signal sample with the plurality of reference non-target signal segments to determine a best non-target match;
- applying metrics to the first signal sample, the best target match, the second signal sample and the best non-target match to determine:
   - whether the classification of the detected first signal segment as a target signal segment is correct; or
   - whether the classification of the detected second signal segment as a non-target signal segment is correct.

In accordance with the present disclosure matching the first signal sample with the plurality of reference target signal segments to determine the best target match can involve for example curve fitting in the time domain of the first signal sample with the plurality of reference target signal segments. In an analogous way, matching the second signal sample with the plurality of reference non-target signal segments to determine the best non-target match can involve for example curve fitting in the time domain of the second signal sample with the plurality of reference non-target signal segments.

In accordance with the present disclosure curve fitting in the time domain may include comparing the signal shape of the first signal sample with the signal shapes of the reference target signal segments of the plurality of reference target signal segments and comparing of the signal shape of the second signal sample with the signal shapes of the reference non-target signal segments of the plurality of reference non-target signal segments. In this case the curve fit resulting in for example the smallest residue can be chosen to determine the best target match and the best non-target match, respectively. However, other aspects related to the curve fitting process can of course also be regarded to determine the best target match and the best non-target match, respectively.

The person skilled in the art will appreciate that curve fitting in the time domain is just an example of the analysis methods that are available to determine the best target match and the best non-target match, respectively. Examples of analysis methods include for example Fast Fourier Transform (FFT), linear signal analysis techniques involving determination of coherence, non-linear signal analysis techniques involving determination of phase synchronization and/or generalized synchronization, template matching, and parametric models including the use of wavelets.

In accordance with the present invention, applying metrics to the first signal sample, the best target match, the second signal sample and the best non-target match can be done in several different ways. A first way of doing this is by establishing and comparing a correlation in the time domain. A second way of doing this is by establishing and comparing a goodness of fit in the wavelet domain. By applying either one of these techniques it can be determined whether the classification of the detected first signal segment as being a target signal segment or the classification of the detected second signal segment as being a non-target signal segment is correct.

As a result of the above, it will be clear that the voting process can eliminate one of the two classifications and thereby will determine the final classification as target signal segment or as non-target signal segment. As a result, false positive classifications or incorrect detections for that matter can be reduced.

In an embodiment of the system according to the invention, the processor is configured and arranged to perform the voting process comprising the process steps of:
- generating a first signal sample that comprises the detected first signal segment;
- matching the first signal sample with a set of reference target signal segments that is based on the plurality of reference target signal segments to determine a best target match;
- generating a second signal sample that comprises the detected second signal segment;
- matching the second signal sample with a set of reference non-target signal segments that is based on the plurality of reference non-target signal segments to determine a best non-target match;
- applying metrics to the first signal sample, the best target match, the second signal sample and the best non-target match to determine:
   - whether the classification of the detected first signal segment as a target signal segment is correct; or
   - whether the classification of the detected second signal segment as a non-target signal segment is correct.

The person skilled in the art will appreciate that the same considerations as mentioned above regarding the previous embodiment of the present invention equally apply to the steps of matching the first signal sample and the second signal sample with the set of reference target signal segments that is based on the plurality of reference target signal samples, and the set of reference non-target signal segments that is based on the plurality of reference non-target signal samples of the currently mentioned embodiment of the present invention.

Moreover, the person skilled in the art will appreciate that the same considerations as mentioned above regarding the previous embodiment of the present invention equally apply to the step of applying metrics to the first signal sample, the best target match, the second signal sample and the best non-target match of the currently mentioned embodiment of the present invention.

Furthermore, it will be clear that by using a set of reference target signal segments that is based on the plurality of reference target signal segments and a set of reference non-target signal segments that is based on the plurality of reference non-target signal segments the voting process can eliminate one of the two classifications without having to use all reference target signal segments of the plurality of reference target signal segments and all reference non-target signal segments of the plurality of reference non-target signal segments, respectively. As a result, the voting process can be performed faster.

In an embodiment of the system according to the invention, the processor is configured and arranged to remove the classification of the detected first signal segment or the classification of the detected second signal segment that based on the voting process is incorrect. The person skilled in the art will appreciate that the voting process results in a so-called winner, i.e. either the classification of the detected first signal segment as being a target signal segment or the classification of the detected second signal segment as being a non-target signal segment is correct. The classification that in accordance with the voting process is to be regarded as incorrect will be removed.

In an embodiment of the system according to the invention, the processor is configured and arranged to apply a predetermined detection boundary that is determined based on the target parameter set and/or the non-target parameter set, the detection boundary allowing a classification of detected signal segments as target signal segments or as non-target signal segments. The detection boundary can for example be a boundary plane in the feature space of wavelet coefficients that is determined by training on a labeled training set of target signal segments and non-target signal segments. In this way, another way to make a distinction between target signal segments and non-target signal segments of a single-channel EEG-recording can be provided. Hence, false positive detections or incorrect classifications for that matter can be reduced.

In an embodiment of the system according to the invention, the processor is configured and arranged to determine an optimized target parameter set that comprises wavelet coefficients that are indicative specifically for the plurality of reference target signal segments and/or an optimized non-target parameter set that comprises wavelet coefficients that are indicative specifically for the plurality of reference non-target signal segments. Determination of the optimized target parameter set and the optimized non-target parameter set can be achieved in several ways. A first way of doing this involves comparing of the wavelet coefficients of the target parameter set and the wavelet coefficients of the non-target parameter set, wherein wavelet coefficients that occur both in the target parameter set and in the non-target parameter set are removed from the target parameter set and/or from the non-target parameter set. In this way, overlap between the target parameter set and the non-target parameter set can be reduced.

A second way of determining the optimized target parameter set and the optimized non-target parameter set involves using the resulting sensitivity and specificity of a system, e.g. a classifier unit, that is adapted to classify a detected first signal segment as being a target signal segment and a detected second signal segment as being a non-target signal segment.

As a result of any one of the above-mentioned first way and second way, the thus optimized target parameter set and non-target parameter set enable an improved distinction between target signal segments and non-target signal segments of a single-channel EEG-recording. Hence, false positive detections or incorrect classifications for that matter can be reduced.

In an embodiment of the system according to the invention, the processor is configured and arranged to apply a predetermined detection boundary that is determined based on the optimized target parameter set and/or the optimized non-target parameter set, the detection boundary allowing an improved classification of detected signal segments as target signal segments or as non-target signal segments. In this way, an improved distinction between target signal segments and non-target signal segments of a single-channel EEG-recording can be achieved. Hence, false positive detections or incorrect classifications for that matter can be reduced.

In an embodiment of the system according to the invention, the system further comprises a data storage unit that is operatively connected to the processor, wherein the data storage unit is configured and arranged to store at least one of the single-channel EEG-recording, the signal obtained from the single-channel EEG-recording, and a classification of a detected signal segment of said signal as a target signal segment or as a non-target signal segment as a result of the method performed by the processor.

In an embodiment of the system according to the invention, the system is configured and arranged to be connectable with two electrodes that are arrangeable on a subject's scalp and are configured to record the single-channel EEG-recording and transfer the single-channel EEG-recording to the data storage unit. The person skilled in the art will appreciate that a system that is connected with more than two electrodes, for example three or four electrodes or any other suitable number, to record the single-channel EEG-recordings also falls within the scope of the present invention as such system is also connected with two electrodes as defined by this exemplary embodiment of the system according to the invention.

### BRIEF DESCRIPTION OF THE DRAWINGS

Further features and advantages of the invention will become apparent from the description of the invention by way of exemplary and non-limiting embodiments of a method for use in the system according to the present invention and a device for use in the system according to the present invention and the system according to the invention for performing the method.

The person skilled in the art will appreciate that the described embodiments of the method for use in the system according to the present invention (the method not being part of the claimed invention) and the device for use in the system according to the present invention, and the system according to the present invention for performing the method are exemplary in nature only and not to be construed as limiting the scope of protection in any way.

Reference will be made to the figures on the accompanying drawing sheets. The figures are schematic in nature and therefore not necessarily drawn to scale. Furthermore, equal reference numerals denote equal or similar parts. On the attached drawing sheets,

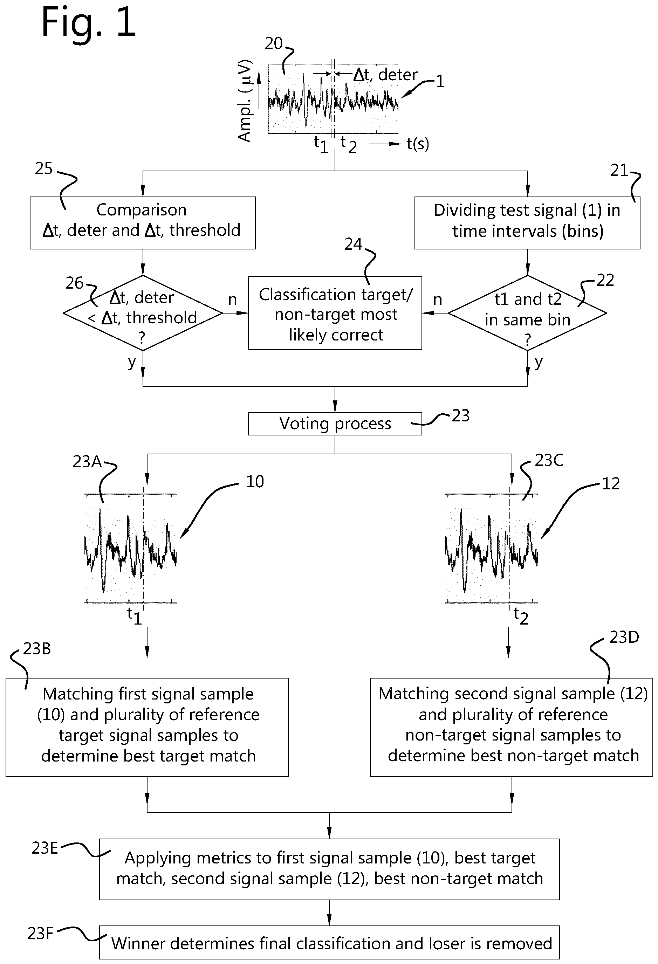

figure 1 shows how a target signal segment of a test signal that is obtained from a single-channel EEG-recording can be classified as a target signal segment or as a non-target signal segment using a system according to the invention For the step of determining a temporal proximity of the first time stamp *t1* that is assigned to a detected first signal segment of the test signal and the second time stamp *t2* that is assigned to a detected second signal segment of the test signal, two exemplary ways of doing this are provided, and

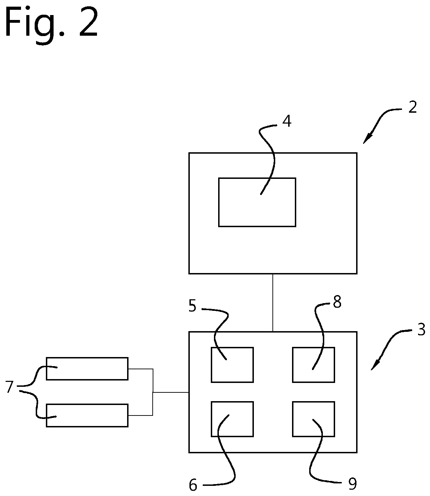

figure 2 shows a schematic layout of exemplary, non-limiting embodiments of a device for use in a system according to the invention and a system according to the invention.

### DETAILED DESCRIPTION OF EMBODIMENTS

Figure 1 shows how a target signal segment of a test signal 1 that is obtained from a single-channel EEG-recording can be classified as a target signal segment or as a non-target signal segment using the system according to the invention The test signal 1 that is schematically represented in figure 1, can be obtained from a single-channel EEG-recording. The detected test signal 1 can be presented to a device 2 for use in a system according to the disclosure and a system 3 according to the invention. Figure 2 shows a schematic layout of exemplary, non-limiting embodiments of the device 2 for use in a system according to the disclosure and the system 3 according to the invention.

In a first step 20 of the method for use in a system according to the invention (the method itself not being part of the claimed invention) the test signal 1 shown in figure 1 is obtained from a single-channel EEG-recording. Single-channel EEG-recordings can be acquired in various known ways including for example using a differential electrode pair, using a single electrode in combination with a reference (REF) electrode or a ground (GND) electrode, or using a differential electrode pair in combination with a REF electrode or a GND electrode. In accordance with the latter exemplary electrode configuration, the individual electrodes of the differential electrode pair can be positioned at specific midline frontal, e.g. Fz, midline vertex, e.g. Cz, midline parietal, e.g. Pz, left medial temporal, e.g. T3, right medial temporal, e.g. T4, positions on a patient's scalp in accordance with an extended 10-20 EEG system with the REF electrode positioned, for example, on an ear of the patient.

The person skilled in the art will appreciate that in the same way single-channel EEG-recordings can be used to obtain a plurality of reference target signal segments and a plurality of reference non-target signal segments.

It is noted that in the context of the present invention target signal segments of a single-channel EEG-recording are to be construed as signal segments that are indicative for a patient being delirious or suffering from related encephalopathy, whereas non-target signal segments of a single-channel EEG-recording are to be construed as signal segments that are indicative for artifacts such as for example eye artifacts, artifacts related to muscle activity, or artifacts related to a combination of such artifacts.

The reference target signal samples of the plurality of reference target signal samples and the reference non-target signal samples of the plurality of reference non-target signal samples can have a predefined duration of for example 15 minutes. However, any suitable predefined duration can be used as long as the acquired single-channel EEG-recordings enable obtaining suitable reference target signal segments and reference non-target signal segments.

The reference target signal segments of the plurality of reference target signal segments can be mutually different. The same holds for the reference non-target signal segments of the plurality of non-target signal segments. The plurality of reference target signal segments can for example comprise more than 1000 reference target signal segments. The same holds for the plurality of non-target signal segments.

In accordance with the method for use in a system according to the invention a target parameter set that is indicative for the plurality of reference target signal segments is applied to the test signal 1 to detect a first signal segment and to classify the detected first signal segment as a target signal segment. In the present example the target parameter set comprises wavelet coefficients that are most representative for the reference target signal segments. The wavelet coefficients have been determined based on the plurality of reference target signal samples using a training process that can involve a machine learning algorithm. The machine learning algorithm can for example use neural networks or deep neural networks.

Upon detecting the first signal segment, a first time stamp *t1* is assigned to it.

Next, a non-target parameter set that is indicative for the plurality of reference non-target signal segments is applied to the same test signal 1 to detect a second signal segment and to classify the detected second signal segment as a non-target signal segment. In the present example the non-target parameter set comprises wavelet coefficients that are most representative for the reference non-target signal segments. The wavelet coefficients have been determined based on the plurality of reference non-target signal samples using another training process that can involve another machine learning algorithm that for example can use neural networks or deep neural networks.

Upon detecting the second signal segment, a second time stamp *t2* is assigned to it.

In an exemplary embodiment of the method for use in a system according to the invention, the wavelet coefficients of the target parameter set and the wavelet coefficients of the non-target parameter set can be compared to optimize the target and non-target parameter sets by removing from either one of them wavelet coefficients that occur in both of them. In this way, overlap between the target parameter set and the non-target parameter set can be reduced. Thus, an optimized target parameter set that comprises wavelet coefficients that are indicative specifically for the plurality of reference target signal segments, and an optimized non-target parameter set that comprises wavelet coefficients that are indicative specifically for the plurality of reference non-target signal segments can be obtained. As a result, the optimized target parameter set and the optimized non-target parameter set enable an improved distinction between target signal segments and non-target signal segments of the single-channel EEG-recording. Hence, false positive detections or incorrect classifications for that matter can be reduced.

In a next step of the method for use in a system according to the present invention (the method itself not being part of the claimed invention) a temporal proximity of the first time stamp *t1* and the second time stamp *t2* is determined. The person skilled in the art will appreciate that the temporal proximity of the first time stamp *t1* and the second time stamp *t2* can be determined in several different ways. Figure 1 shows two exemplary ways of doing this.

A first exemplary way of doing this that is explained in relation to step 21 in figure 1, involves dividing the obtained test signal 1 in time intervals of a predefined length. These time intervals can be referred to as bins. Preferably, the time intervals or bins have a predefined equal length. The predefined length of the time intervals can be chosen depending on specific requirements such as desired accuracy. Suitable predefined lengths of the time intervals range between 0.25 s and 3 s. Preferably, the time intervals have a predefined length of 1 s. Determining the temporal proximity of the first time stamp *t1* and the second time stamp *t2* is based on establishing if the first time stamp *t1* and the second time stamp *t2* fall within the same time interval or not. This is indicated as step 22 in figure 1. If the first time stamp *t1* and the second time stamp *t2* fall within the same time interval or bin, then the voting process, which is indicated as step 23 in figure 1, is required and will be performed to determine whether classification of the detected first signal segment as a target signal segment or classification of the detected second signal segment as a non-target signal segment is correct. If the first time stamp *t1* and the second time stamp *t2* fall in different time intervals, i.e. the first time stamp *t1* and the second time stamp *t2* do not fall within the same bin, then the voting process is not required and preferably the voting process is not performed. In this case, the classification of the detected first signal segment as being a target signal segment and the classification of the detected second signal segment as being a non-target signal segment are most likely both correct. This is indicated as step 24 in figure 1.

A second way of determining the temporal proximity of the first time stamp *t1* and the second time stamp *t2* that is explained in relation to step 25 in figure 1, according to the invention, involves determining a time difference *Δt,deter* between the first time stamp *t1* and the second time stamp *t2.* The determined time difference *Δt,deter* is compared with a predefined threshold *Δt,threshold.* The person skilled in the art will appreciate that the threshold *Δt,threshold* is chosen such that when the determined time difference *Δt,deter* is smaller than the threshold *Δt,threshold,* it is not likely or not possible at all that the classification of the detected first signal segment is assigned as being a target signal segment and the classification of the detected second signal segment is assigned as being a non-target signal segment can both be correct. Hence, as indicated in step 26 in figure 1, when the determined time difference *Δt,deter* is smaller than the threshold *Δt,threshold,* then a voting process, which is indicated as step 23 in figure 1, will be performed to determine whether classification of the detected first signal segment as a target signal segment or the classification of the detected second signal segment as a non-target signal segment is correct.

However, if the determined time difference *Δt,deter* is equal to or larger than the threshold *Δt,threshold,* then the classification of the detected first signal segment as being a target signal segment and the classification of the detected second signal segment as being a non-target signal segment are most likely both correct. This is indicated as step 24 in figure 1.

The person skilled in the art will appreciate that any suitable threshold *Δt,threshold* can be chosen as long as it allows to establish whether the classification of the detected first signal segment as being a target signal segment and/or the classification of the detected second signal segment as being a non-target signal segment can be correct. Suitable values for the predefined threshold range between 0.25 s and 3 s. Preferably, the threshold is 1 s. Based on the above, it will be clear that the voting process eliminates one of the classifications. As a result, the method for use in a system according to the present invention can reduce false positive detections or incorrect classifications for that matter.

The voting process of the method for use in a system of the present invention (the method itself not being part of the claimed invention), comprises a step 23A of generating a first signal sample 10 that comprises the detected first signal segment to which the first time stamp *t1* has been assigned.

In a next step 23B of the voting process the generated first signal sample 10 is matched with the plurality of reference target signal segments to determine a best target match.

In a similar way, another step 23C in the voting process is generating a second signal sample 12 that comprises the detected second signal segment to which the second time stamp *t2* has been assigned. Then, in a next step 23D of the voting process, the second signal sample 12 is matched with the plurality of reference non-target signal segments to determine a best non-target match.

In accordance with the present invention, matching the first signal sample 10 with the plurality of reference target signal segments to determine the best target match can involve for example curve fitting in the time domain of the first signal sample 10 with the plurality of reference target signal segments. In an analogous way, matching the second signal sample 12 with the plurality of reference non-target signal segments to determine the best non-target match can involve for example curve fitting in the time domain of the second signal sample 12 with the plurality of reference non-target signal segments. Curve fitting in the time domain may include comparing the signal shape of the first signal sample 10 with the signal shapes of the reference target signal segments of the plurality of reference target signal segments and comparing of the signal shape of the second signal sample 12 with the signal shapes of the reference non-target signal segments of the plurality of reference non-target signal segments. In this case, the curve fit resulting in for example the smallest residue can be chosen to determine the best target match and the best non-target match, respectively. However, other aspects related to the curve fitting process can of course also be regarded to determine the best target match and the best non-target match, respectively.

The person skilled in the art will appreciate that curve fitting in the time domain is just an example of the analysis methods that are available to determine the best target match and the best non-target match, respectively. Examples of analysis methods include for example Fast Fourier Transform (FFT), linear signal analysis techniques involving determination of coherence, non-linear signal analysis techniques involving determination of phase synchronization and/or generalized synchronization, template matching, and parametric models including the use of wavelets.

As a next step 23E of the voting process, metrics are applied to the first signal sample 10, the best target match, the second signal sample 12 and the best non-target match parameter to determine whether the classification of the detected first signal segment as a target signal segment is correct, or whether the classification of the detected second signal segment as a non-target signal segment is correct.

In accordance with the present invention, applying metrics to the first signal sample 10, the best target match, the second signal sample 12 and the best non-target match can be done in several different ways. A first way of doing this is by establishing and comparing a correlation in the time domain. A second way of doing this is by establishing and comparing a goodness of fit in the wavelet domain. By applying either one of these techniques it can be determined whether the classification of the detected first signal segment as being a target signal segment or the classification of the detected second signal segment as being a non-target signal segment is correct.

Based on the above it will be clear that the voting process of the method for use in a system of the present invention will result in a so-called winner, i.e. the voting process eliminates one of the two classifications and thereby will determine the final classification as target signal segment or as non-target signal segment. As a result, false positive classifications or incorrect detections for that matter can be reduced. The loser is removed. This is indicated as step 23F in figure 1.

Figure 2 shows a schematic layout of exemplary, non-limiting embodiments of a device 2 for use in a system according to the disclosure and a system 3 according to the invention. The device 2 can be construed as a detector that is configured and arranged to be used with the system 3 that is configured and arranged to detect and classify a segment of a signal that is obtained from a single-channel EEG-recording as a target signal segment or as a non-target signal segment. The device 2 has a database 4 that comprises at least one of a plurality of reference target signal segments that are obtained from signals that are obtained from reference single-channel EEG-recordings, a set of reference target signal segments that is based on the plurality of reference target signal segments, a plurality of reference non-target signal segments that are obtained from signals that are obtained from reference single-channel EEG-recordings, a set of reference non-target signal segments that is based on the plurality of reference non-target signal segments, a target parameter set that is indicative for the plurality of reference target signal segments, and a non-target parameter set that is indicative for a plurality of reference non-target signal segments. As mentioned above, the target parameter set and the non-target parameter set can be obtained via respective training processes that may involve respective machine learning algorithms that may use respective deep neural networks.

The device 2 for use in a system according to the invention enables an improved distinction between target signal segments and non-target signal segments of a signal that is obtained from a single-channel EEG-recording. As a result, false positive detections or incorrect classifications for that matter as discussed above can be reduced.

The system 3 according to the invention is configured and arranged to detect and classify a segment of a signal that is obtained from a single-channel EEG-recording as a target signal segment or as a non-target signal segment in accordance with the method for use in a system of the present invention (the method itself not being part of the claimed invention). The system 3 comprises a processor 5 that is configured and arranged to perform the method for use in a system according to the present invention on said signal when being operatively connected to the device 2 for use in a system according to the present disclosure.

In this way, the system 3 and the device 2 when being operatively connected can be used to achieve an improved distinction between target signal segments and non-target signal segments of a signal that is obtained from a single-channel EEG-recording. As a result, false positive detections or incorrect classifications for that matter as discussed above can be reduced. The person skilled in the art will appreciate that the device 2 and the system 3 can be implemented as separate units as is schematically shown in figure 2. However, the device 2 and the system 3 can also be implemented as an integrated unit (not shown).

The system 3 shown in figure 2 further comprises a data storage unit 6 that is operatively connected to the processor 5. The data storage unit 6 can be configured and arranged to store at least one of the single-channel EEG-recording, the signal obtained from the single-channel EEG-recording, and a classification of a detected signal segment of said signal as a target signal segment or as a non-target signal segment as a result of the method performed by the processor 5.

In the exemplary, non-limiting embodiment of the system 3 shown in figure 2, the system 3 is connected with two electrodes 7 that are arrangeable on a subject's scalp and are configured to record the single-channel EEG-recordings and transfer the single-channel EEG-recordings to the data storage unit 6. The system 3 can further be configured to comprise application software 8 and a display unit 9, such as a screen.

The present invention can be summarized as relating to a system, which system uses a method for detecting and classifying a segment of a signal 1 that is obtained from an EEG-recording as a target signal segment or as a non-target signal segment. The method (the method itself not being part of the claimed invention) comprises a voting process to determine whether classification of a first detected segment of the signal as a target signal segment or classification of a second detected segment of the signal as a non-target signal segment is correct. The invention relates to a system 3 as defined in the appended claims.

It will be clear to a person skilled in the art that the scope of the present invention is not limited to the examples discussed in the foregoing but that several amendments and modifications thereof are possible without deviating from the scope of the present invention as defined by the attached claims. In particular, combinations of specific features of various aspects of the invention may be made. An aspect of the invention may be further advantageously enhanced by adding a feature that was described in relation to another aspect of the invention. While the present invention has been illustrated and described in detail in the figures and the description, such illustration and description are to be considered illustrative or exemplary only, and not restrictive.

## Claims

1. A system (3) that is configured and arranged to detect and classify a segment of a signal (1) that is obtained from a single-channel EEG-recording as a target signal segment or as a non-target signal segment, the system (3) comprising a processor (5) that is configured and arranged to perform on said signal (1), when being operatively connected to a device (2) having a database (4), the process steps of:
• providing a signal (1) that is obtained from a single-channel EEG-recording;
• applying to said signal (1) a target parameter set, which is indicative for a plurality of reference target signal segments that are obtained from reference single channel EEG-recordings, to detect a first signal segment of said signal (1) and to classify the detected first signal segment as a target signal segment, wherein the target parameter set comprises wavelet coefficients that are determined using wavelet decomposition of the plurality of reference target signal segments;
• assigning a first time stamp (t1) to the detected first signal segment;
• applying to said signal (1) a non-target parameter set, which is indicative for a plurality of reference non-target signal segments that are obtained from reference single-channel EEG-recordings, to detect a second signal segment of said signal (1) and to classify the detected second signal segment as a non-target signal segment, wherein the non-target parameter set comprises wavelet coefficients that are determined using wavelet decomposition of the plurality of reference non-target signal segments;
• assigning a second time stamp (t2) to the detected second signal segment;
• determining a time difference between the first time stamp (t1) and the second time stamp (t2);
• when said determined time difference is smaller than a predetermined threshold, determining if a voting process is required to determine whether classification of the detected first signal segment as a target signal segment or classification of the detected second signal segment as a non-target signal segment is correct; and
• upon establishing that said voting process is required, performing said voting process;
wherein the database (4) comprises at least one of:
• the plurality of reference target signal segments that are obtained from reference single-channel EEG-recordings;
• a set of reference target signal segments that is based on the plurality of reference target signal segments;
• the plurality of reference non-target signal segments that are obtained from reference single-channel EEG-recordings;
• a set of reference non-target signal segments that is based on the plurality of reference non-target signal segments;
• the target parameter set that is indicative for the plurality of reference target signal segments, wherein the target parameter set comprises wavelet coefficients that are determined using wavelet decomposition of the plurality of reference target signal segments; and
• the non-target parameter set that is indicative for a plurality of reference non-target signal segments, wherein the non-target parameter set comprises wavelet coefficients that are determined using wavelet decomposition of the plurality of reference non-target signal segments.

2. The system according to claim 1,
• wherein, in the step of applying to said signal (1) the target parameter set, the detected first signal segment is classified as a target signal segment when the detected first signal segment is indicative for a patient being delirious or suffering from related encephalopathy, and
• wherein, in the step of applying to said signal (1) the non-target parameter set, the detected second signal segment is classified as a non-target signal segment when the detected second signal segment is indicative for artifacts.

3. The system (3) according to claim 1 or 2, wherein the processor (5) is configured and arranged to perform the voting process comprising either the process steps of:
• generating a first signal sample (10) that comprises the detected first signal segment;
• matching the first signal sample (10) with the plurality of reference target signal segments to determine a best target match;
• generating a second signal sample (12) that comprises the detected second signal segment;
• matching the second signal sample with the plurality of reference non-target signal segments to determine a best non-target match;
• applying metrics to the first signal sample (10), the best target match, the second signal sample (12) and the best non-target match to determine:
- whether the classification of the detected first signal segment as a target signal segment is correct; or
- whether the classification of the detected second signal segment as a non-target signal segment is correct;
or the process steps of:
• generating a first signal sample (10) that comprises the detected first signal segment;
• matching the first signal sample (10) with a set of reference target signal segments that is based on the plurality of reference target signal segments to determine a best target match;
• generating a second signal sample (12) that comprises the detected second signal segment;
• matching the second signal sample (12) with a set of reference non-target signal segments that is based on the plurality of reference non-target signal segments to determine a best non-target match;
• applying metrics to the first signal sample (10), the best target match, the second signal sample (12) and the best non-target match to determine:
- whether the classification of the detected first signal segment as a target signal segment is correct; or
- whether the classification of the detected second signal segment as a non-target signal segment is correct.

4. The system (3) according to any one of the claims 1-3 , wherein the processor (5) is configured and arranged:
• to remove the classification of the detected first signal segment or the classification of the detected second signal segment that based on the voting process is incorrect;
and/or
to apply a predetermined detection boundary that is determined based on the target parameter set and/or the non-target parameter set, the detection boundary allowing a classification of detected signal segments as target signal segments or as non-target signal segments.

5. The system (3) according to any one of the claims 1-4, wherein the processor (5) is configured and arranged to determine an optimized target parameter set that comprises wavelet coefficients that are indicative specifically for the plurality of reference target signal segments and/or an optimized non-target parameter set that comprises wavelet coefficients that are indicative specifically for the plurality of reference non-target signal segments.

6. The system (3) according to claim 5, wherein the processor (5) is configured and arranged to apply a predetermined detection boundary that is determined based on the optimized target parameter set and/or the optimized non-target parameter set, the detection boundary allowing an improved classification of detected signal segments as target signal segments or as non-target signal segments.

7. The system (3) according to any one of the claims 1-6, further comprising a data storage unit (6) that is operatively connected to the processor (5), wherein the data storage unit (6) is configured and arranged to store at least one of the single-channel EEG-recording and the signal obtained from the single-channel EEG-recording, and a classification of a detected signal segment of said signal as a target signal segment or as a non-target signal segment as a result of the method performed by the processor (5).

8. The system (3) according to claim 7, wherein the system (3) is configured and arranged to be connectable with two electrodes (7) that are arrangeable on a subject's scalp and are configured to record the single-channel EEG-recording and transfer the single-channel EEG-recording to the data storage unit (6).

## Patentansprüche

1. System (3), das konfiguriert und angeordnet ist, um ein Segment eines Signals (1) zu erfassen und zu klassifizieren, das aus einer Einkanal-EEG-Aufzeichnung als ein Zielsignalsegment oder als ein Nichtzielsignalsegment erhalten wird, das System (3) umfassend einen Prozessor (5), der konfiguriert und angeordnet ist, um auf dem Signal (1) durchzuführen, wenn er mit einer Vorrichtung (2), die eine Datenbank (4) aufweist, wirkverbunden ist, die Prozessschritte zum:
• Bereitstellen eines Signals (1), das aus einer Einkanal-EEG-Aufzeichnung erhalten wird;
• Anwenden eines Zielparametersatzes auf das Signal (1), der eine Vielzahl von Referenzzielsignalsegmenten angibt, die aus Referenz-Einkanal-EEG-Aufzeichnungen erhalten wird, um ein erstes Signalsegment des Signals (1) zu erfassen und um das erfasste erste Signalsegment als ein Zielsignalsegment zu klassifizieren, wobei der Zielparametersatz Wavelet-Koeffizienten umfasst, die unter Verwendung von Wavelet-Zerlegung der Vielzahl von Referenzzielsignalsegmenten bestimmt werden;
• Zuweisen eines ersten Zeitstempels (t1) zu dem erfassten ersten Signalsegment;
• Anwenden eines Nichtzielparametersatzes auf das Signal (1), der eine Vielzahl von Referenznichtzielsignalsegmenten angibt, die aus Referenz-Einkanal-EEG-Aufzeichnungen erhalten wird, um ein zweites Signalsegment des Signals (1) zu erfassen und um das erfasste zweite Signalsegment als ein Nichtzielsignalsegment zu klassifizieren, wobei der Nichtzielparametersatz Wavelet-Koeffizienten umfasst, die unter Verwendung von Wavelet-Zerlegung der Vielzahl von Referenznichtzielsignalsegmenten bestimmt werden;
• Zuweisen eines zweiten Zeitstempels (t2) zu dem erfassten zweiten Signalsegment;
• Bestimmen einer Zeitdifferenz zwischen dem ersten Zeitstempel (t1) und dem zweiten Zeitstempel (t2);
• wenn die bestimmte Zeitdifferenz kleiner als ein zuvor bestimmter Schwellenwert ist, Bestimmen, ob ein Stimmprozess erforderlich ist, um zu bestimmen, ob eine Klassifizierung des erfassten ersten Signalsegments als ein Zielsignalsegment oder eine Klassifizierung des erfassten zweiten Signalsegments als ein Nichtzielsignalsegment korrekt ist; und
• nach einem Einrichten, dass der Stimmprozess erforderlich ist, Durchführen des Stimmprozesses;
wobei die Datenbank (4) mindestens eines umfasst von:
• der Vielzahl von Referenzzielsignalsegmenten, die aus Referenz-Einkanal-EEG-Aufzeichnungen erhalten wird;
• einem Satz von Referenzzielsignalsegmenten, der auf der Vielzahl von Referenzzielsignalsegmenten basiert;
• der Vielzahl von Referenznichtzielsignalsegmenten, die aus Referenz-Einkanal-EEG-Aufzeichnungen erhalten wird;
• einem Satz von Referenznichtzielsignalsegmenten, die auf der Vielzahl von Referenznichtzielsignalsegmenten basiert;
• dem Zielparametersatz, der die Vielzahl von Referenzzielsignalsegmenten angibt, wobei der Zielparametersatz Wavelet-Koeffizienten umfasst, die unter Verwendung von Wavelet-Zerlegung der Vielzahl von Referenzzielsignalsegmenten bestimmt werden; und
• dem Nichtzielparametersatz, der eine Vielzahl von Referenznichtzielsignalsegmenten angibt, wobei der Nichtzielparametersatz Wavelet-Koeffizienten umfasst, die unter Verwendung von Wavelet-Zerlegung der Vielzahl von Referenznichtzielsignalsegmenten bestimmt werden.

2. System nach Anspruch 1,
• wobei, in dem Schritt des Anwendens des Zielparametersatzes auf das Signal (1), das erfasste erste Signalsegment als ein Zielsignalsegment klassifiziert wird, wenn das erfasste erste Signalsegment einen Patienten angibt, der delirant ist oder an verwandter Enzephalopathie leidet, und
• wobei, in dem Schritt des Anwendens (1) des Nichtzielparametersatzes auf das Signal, das erfasste zweite Signalsegment als ein Nichtzielsignalsegment klassifiziert wird, wenn das erfasste zweite Signalsegment Artefakte angibt.

3. System (3) nach Anspruch 1 oder 2, wobei der Prozessor (5) konfiguriert und angeordnet ist, um den Stimmprozess durchzuführen, umfassend entweder die Prozessschritte zum:
• Erzeugen einer ersten Signalprobe (10), die das erfasste erste Signalsegment umfasst;
• Übereinstimmen der ersten Signalprobe (10) mit der Vielzahl von Referenzzielsignalsegmenten, um eine beste Zielübereinstimmung zu bestimmen;
• Erzeugen einer zweiten Signalprobe (12), die das erfasste zweite Signalsegment umfasst;
• Übereinstimmen der zweiten Signalprobe mit der Vielzahl von Referenznichtzielsignalsegmenten, um eine beste Nichtzielübereinstimmung zu bestimmen;
• Anwenden von Metriken auf die erste Signalprobe (10), die beste Zielübereinstimmung, die zweite Signalprobe (12) und die beste Nichtzielübereinstimmung, um zu bestimmen:
- ob die Klassifizierung des erfassten ersten Signalsegments als ein Zielsignalsegment korrekt ist; oder
- ob die Klassifizierung des erfassten zweiten Signalsegments als ein Nichtzielsignalsegment korrekt ist;
oder die Prozessschritte zum:
• Erzeugen einer ersten Signalprobe (10), die das erfasste erste Signalsegment umfasst;
• Übereinstimmen der ersten Signalprobe (10) mit einem Satz von Referenzzielsignalsegmenten, die auf der Vielzahl von Referenzzielsignalsegmenten basieren, um eine beste Zielübereinstimmung zu bestimmen;
• Erzeugen einer zweiten Signalprobe (12), die das erfasste zweite Signalsegment umfasst;
• Übereinstimmen der zweiten Signalprobe (12) mit einem Satz von Referenznichtzielsignalsegmenten, die auf der Vielzahl von Referenznichtzielsignalsegmenten basieren, um eine beste Nichtzielübereinstimmung zu bestimmen;
• Anwenden von Metriken auf die erste Signalprobe (10), die beste Zielübereinstimmung, die zweite Signalprobe (12) und die beste Nichtzielübereinstimmung, um zu bestimmen:
- ob die Klassifizierung des erfassten ersten Signalsegments als ein Zielsignalsegment korrekt ist; oder
- ob die Klassifizierung des erfassten zweiten Signalsegments als ein Nichtzielsignalsegment korrekt ist.

4. System (3) nach einem der Ansprüche 1 bis 3, wobei der Prozessor (5) konfiguriert und angeordnet ist:
• zum Entfernen der Klassifizierung des erfassten ersten Signalsegments oder der Klassifizierung des erfassten zweiten Signalsegments, das basierend auf dem Stimmprozess nicht korrekt ist;
und/oder
zum Anwenden einer zuvor bestimmten Erfassungsgrenze, die basierend auf dem Zielparametersatz und/oder dem Nichtzielparametersatz bestimmt wird, wobei die Erfassungsgrenze eine Klassifizierung von erfassten Signalsegmenten als Zielsignalsegmente oder als Nichtzielsignalsegmente ermöglicht.

5. System (3) nach einem der Ansprüche 1 bis 4, wobei der Prozessor (5) konfiguriert und angeordnet ist, um einen optimierten Zielparametersatz zu bestimmen, der Wavelet-Koeffizienten umfasst, die speziell für die Vielzahl von Referenzzielsignalsegmenten und/oder einen optimierten Nichtzielparametersatz angeben, der Wavelet-Koeffizienten umfasst, die speziell für die Vielzahl von Referenznichtzielsignalsegmenten angeben.

6. System (3) nach Anspruch 5, wobei der Prozessor (5) konfiguriert und angeordnet ist, um eine zuvor bestimmte Erfassungsgrenze anzuwenden, die basierend auf dem optimierten Zielparametersatz und/oder dem optimierten Nichtzielparametersatz bestimmt wird, wobei die Erfassungsgrenze eine verbesserte Klassifizierung von erfassten Signalsegmenten als Zielsignalsegmente oder als Nichtzielsignalsegmente ermöglicht.

7. System (3) nach einem der Ansprüche 1 bis 6, ferner umfassend eine Datenspeicherungseinheit (6), die mit dem Prozessor (5) wirkverbunden ist, wobei die Datenspeicherungseinheit (6) konfiguriert und angeordnet ist, um mindestens eines von der Einkanal-EEG-Aufzeichnung und dem Signal, das von der Einkanal-EEG-Aufzeichnung erhalten wird, und einer Klassifizierung eines erfassten Signalsegments des Signals als ein Zielsignalsegment oder als ein Nichtzielsignalsegment als ein Ergebnis des Verfahrens zu speichern, das durch den Prozessor (5) durchgeführt wird.

8. System (3) nach Anspruch 7, wobei das System (3) konfiguriert und angeordnet ist, um mit zwei Elektroden (7) verbindbar zu sein, die an einer Kopfhaut des Subjekts anordenbar sind und konfiguriert sind, um die Einkanal-EEG-Aufzeichnung aufzuzeichnen und die Einkanal-EEG-Aufzeichnung an die Datenspeicherungseinheit (6) zu übertragen.

## Revendications

1. Système (3) qui est configuré et agencé pour détecter et classifier un segment d'un signal (1) qui est obtenu à partir d'un enregistrement EEG à canal unique en tant que segment de signal cible ou en tant que segment de signal non cible, le système (3) comprenant un processeur (5) qui est configuré et agencé pour réaliser sur ledit signal (1), lorsqu'il est connecté fonctionnellement à un dispositif (2) présentant une base de données (4), les étapes de processus consistant à :
• fournir un signal (1) qui est obtenu à partir d'un enregistrement EEG à canal unique ;
• appliquer audit signal (1) un ensemble de paramètres cible, qui indique une pluralité de segments de signal cibles de référence qui sont obtenus à partir des enregistrements EEG à canal unique de référence, pour détecter un premier segment de signal dudit signal (1) et pour classifier le premier segment de signal détecté en tant que segment de signal cible, dans lequel l'ensemble de paramètres cible comprend des coefficients d'ondelettes qui sont déterminés à l'aide d'une décomposition en ondelettes de la pluralité de segments de signal cible de référence ;
• attribuer un premier horodatage (t1) au premier segment de signal détecté ;
• appliquer audit signal (1) un ensemble de paramètres non cible, qui indique une pluralité de segments de signal non cibles de référence qui sont obtenus à partir des enregistrements EEG à canal unique de référence, pour détecter un second segment de signal dudit signal (1) et pour classifier le second segment de signal détecté en tant que segment de signal non cible, dans lequel l'ensemble de paramètres non cible comprend des coefficients d'ondelettes qui sont déterminés à l'aide d'une décomposition en ondelettes de la pluralité de segments de signal non cibles de référence ;
• attribuer un second horodatage (t2) au second segment de signal détecté ;
• déterminer une différence temporelle entre le premier horodatage (t1) et le second horodatage (t2) ;
• lorsque ladite différence temporelle déterminée est inférieure à un seuil prédéterminé, déterminer si un processus de vote est requis pour déterminer si une classification du premier segment de signal détecté en tant que segment de signal cible ou une classification du second segment de signal détecté en tant que segment de signal non cible est correcte ; et
• s'il est établi que ledit processus de vote est requis, la réalisation dudit processus de vote ;
dans lequel la base de données (4) comprend au moins l'un parmi :
• la pluralité de segments de signal cibles de référence qui sont obtenus à partir d'enregistrements d'EEG à canal unique de référence ;
• un ensemble de segments de signal cibles de référence qui est basé sur la pluralité de segments de signal cible de référence ;
• la pluralité de segments de signal non cibles de référence qui sont obtenus à partir d'enregistrements d'EEG à canal unique de référence ;
• un ensemble de segments de signal non cibles de référence qui est basé sur la pluralité de segments de signal non cible de référence ;
• l'ensemble de paramètres cible qui indique la pluralité de segments de signal cibles de référence, dans lequel l'ensemble de paramètres cible comprend des coefficients d'ondelettes qui sont déterminés à l'aide d'une décomposition en ondelettes de la pluralité de segments de signal cibles de référence ; et
• l'ensemble de paramètres non cible qui indique une pluralité de segments de signal non cibles de référence, dans lequel l'ensemble de paramètres non cible comprend des coefficients d'ondelettes qui sont déterminés à l'aide d'une décomposition en ondelettes de la pluralité de segments de signal non cible de référence.

2. Système selon la revendication 1,
• dans lequel, lors de l'étape d'application audit signal (1) l'ensemble de paramètres cible, le premier segment de signal détecté est classifié en tant que segment de signal cible lorsque le premier segment de signal détecté indique un patient déflagrant ou souffrant d'une encéphalopathie liée, et
• dans lequel, lors de l'étape d'application audit signal (1) de l'ensemble de paramètres non cible, le second segment de signal détecté est classifié comme un segment de signal non cible lorsque le second segment de signal détecté indique des artéfacts.

3. Système (3) selon la revendication 1 ou 2, dans lequel le processeur (5) est configuré et agencé pour réaliser le processus de vote comprenant
soit les étapes de processus consistant à :
• générer un premier échantillon de signal (10) qui comprend le premier segment de signal détecté ;
• mettre en correspondance le premier échantillon de signal (10) avec la pluralité de segments de signal cibles de référence pour déterminer une meilleure correspondance cible ;
• générer un second échantillon de signal (12) qui comprend le second segment de signal détecté ;
• mettre en correspondance le second échantillon de signal avec la pluralité de segments de signal non cibles de référence pour déterminer une meilleure correspondance non cible ;
• appliquer des mesures au premier échantillon de signal (10), à la meilleure correspondance cible, au second échantillon de signal (12) et à la meilleure correspondance non cible pour déterminer :
- si la classification du premier segment de signal détecté en tant que segment de signal cible est correcte ; ou
- si la classification du second segment de signal détecté en tant que segment de signal non cible est correcte ;
ou les étapes de processus consistant à :
• générer un premier échantillon de signal (10) qui comprend le premier segment de signal détecté ;
• mettre en correspondance le premier échantillon de signal (10) avec un ensemble de segments de signal cibles de référence qui est basé sur la pluralité de segments de signal cibles de référence pour déterminer une meilleure correspondance cible ;
• générer un second échantillon de signal (12) qui comprend le second segment de signal détecté ;
• mettre en correspondance le second échantillon de signal (12) avec un ensemble de segments de signal non cibles de référence qui est basé sur la pluralité de segments de signal non cibles de référence pour déterminer une meilleure correspondance non cible ;
• appliquer des mesures au premier échantillon de signal (10), à la meilleure correspondance cible, au second échantillon de signal (12) et à la meilleure correspondance non cible pour déterminer :
- si la classification du premier segment de signal détecté en tant que segment de signal cible est correcte ; ou
- si la classification du second segment de signal détecté en tant que segment de signal non cible est correcte.

4. Système (3) selon l'une quelconque des revendications 1 à 3, dans lequel le processeur (5) est configuré et agencé :
• pour supprimer la classification du premier segment de signal détecté ou la classification du second segment de signal détecté qui est incorrecte sur la base du processus de vote ;
et/ou
pour appliquer une limite de détection prédéterminée qui est déterminée sur la base de l'ensemble de paramètres cible et/ou de l'ensemble de paramètres non cible, la limite de détection permettant une classification de segments de signal détectés en tant que segments de signal cibles ou en tant que segments de signal non cibles.

5. Système (3) selon l'une quelconque des revendications 1 à 4, dans lequel le processeur (5) est configuré et agencé pour déterminer un ensemble de paramètres cible optimisé qui comprend des coefficients d'ondelette qui indiquent spécifiquement la pluralité de segments de signal cibles de référence et/ou un ensemble de paramètres non cible optimisé qui comprend des coefficients d'ondelette qui indiquent spécifiquement la pluralité de segments de signal non cible de référence.

6. Système (3) selon la revendication 5, dans lequel le processeur (5) est configuré et agencé pour appliquer une limite de détection prédéterminée qui est déterminée sur la base de l'ensemble de paramètres cible optimisé et/ou de l'ensemble de paramètres non cible optimisé, la limite de détection permettant une classification améliorée de segments de signal détectés en tant que segments de signal cibles ou en tant que segments de signal non cibles.

7. Système (3) selon l'une quelconque des revendications 1 à 6, comprenant en outre une unité de stockage de données (6) qui est fonctionnellement connectée au processeur (5), dans lequel l'unité de stockage de données (6) est configurée et agencée pour stocker au moins l'un parmi l'enregistrement EEG à canal unique et le signal obtenu à partir de l'enregistrement d'EEG à canal unique, et une classification d'un segment de signal détecté dudit signal en tant que segment de signal cible ou en tant que segment de signal non cible comme résultat du procédé réalisé par le processeur (5).

8. Système (3) selon la revendication 7, dans lequel le système (3) est configuré et agencé pour être connectable à deux électrodes (7) qui peuvent être agencées sur le cuir chevelu d'un sujet et sont configurées pour enregistrer l'enregistrement d'EEG à canal unique et transférer l'enregistrement d'EEG à canal unique à l'unité de stockage de données (6).
